# EUROPEAN PATENT APPLICATION

(11) **EP 1 854 396 A1**
(43) Date of publication of application: **14.11.2007**
(21) Application number: 06715086.2
(22) Date of filing: 02.03.2006
(51) Int. Cl.: A61B 1/00, A61B 5/0408, A61B 5/0478, A61B 5/07

(54) **DETECTION DEVICE AND METHOD OF PRODUCING DETECTION DEVICE**

(30) Priority: 03.03.2005 JP 2005059558
(71) Applicant: OLYMPUS MEDICAL SYSTEMS CORP., Shibuya-ku Tokyo 151-0072 (JP)
(72) Inventor: NAGASE, Ayako;c/o OLYMPUS MED. SYSTEMS CORP., Tokyo 151-0072 (JP)
(74) Representative: von Hellfeld, Axel
(86) International application number: PCT/JP2006/303977
(87) International publication number: WO 2006/093223

(57) **Abstract**

To provide a detecting device that is capable of securing flexibility in mounting and after mounting detectors such as antennas, and a manufacturing method of the detecting device. In the detecting device, a plurality of receiving antennas A11 to A18 and a radio unit are connected through a plurality of coaxial cables 10-1 to 10-8, and the plural and independent coaxial cables 10-1 to 10-8 are fixed at predetermined intervals with cable clips 20a to 20d in parallel, on a side of the radio unit, thereby being formed into a flat cable.

## Description

### TECHNICAL FIELD

The present invention relates to a detecting device in which a plurality of detectors and a processing device that receives and processes a detection signal from the plurality of detectors are connected through a plurality of cables, and a manufacturing method of the detecting device.

### BACKGROUND ART

Recently, in the field of endoscope, a swallowable capsule endoscope has appeared. In this capsule endoscope, an imaging function and a radio communication function are provided. The capsule endoscope has a function of sequentially taking images after the capsule endoscope is swallowed from the mouth of a patient for observation (examination) until naturally discharged out of a human body, while passing through body cavities, for example, inside of organs such as a stomach and a small intestine, according to the peristalsis thereof.

Image data obtained inside the body by the capsule endoscope while moving inside the body cavities is sequentially transmitted to the outside by radio communication, and stored in a memory provided in an external receiver. If the patient carries the receiver having the radio communication function and the memory function, the patient can freely move even during the period from swallowing the capsule endoscope until it is discharged. Doctors and nurses can perform diagnosis based on the image data stored in the memory by displaying images of the organs on a display.

Patent Document 1: Japanese Patent Application Laid-open No. 2003-19111

### DISCLOSURE OF INVENTION

### PROBLEM TO BE SOLVED BY THE INVENTION

The above described receiver serving as a detecting device includes antennas serving as a plurality of detectors that are arranged at desirable positions on the outside-body of a patient, and the antennas and the receiver are connected with a plurality of coaxial cables. Since mounting becomes difficult due to free movement of the plural coaxial cables, there has been a technique in which the respective antennas are prevented from scattering by forming the coaxial cables into a flat cable by wrapping with a one-piece outer layer such that this flat cable sequentially branches off to the antennas.

However, when the antennas are mounted on the outside-body of a patient using this flat cable, since each coaxial cable is provided in a form of a flat cable, movement of the cables in a lateral direction, which is a direction perpendicular to a longitudinal direction of the coaxial cables, is limited. Therefore, there has been a problem in which flexibility in mounting the antennas is limited and movement of the patient is restricted.

The present invention has been achieved in view of the above problems, and it is an object of the present invention to provide a detecting device capable of securing flexibility at the time of mounting and after mounting the detectors such as antennas, and a manufacturing method of the detecting device.

### MEANS FOR SOLVING PROBLEM

To solve the above problems and to achieve an object, a detecting device in which a plurality of detectors and a processing device which receives and processes a detection signal from the detectors are connected through a plurality of cables, according to claim 1, includes a cable clip that forms the plurality of respective cables into a flat cable so that the cables which are placed parallel to and in contact with each other are restrained from moving at desirable positions from a side of the processing device.

In the detecting device according to the invention as set forth in claim 2, the cables are coaxial cables, and the cable clip is arranged so as to make the coaxial cables branch off in stages by decreasing a number of the coaxial cables which is formed into the flat cable in stages from the side of the processing device.

In the detecting device according to the invention as set forth in claim 3, a length of the cables formed into the flat cable with the cable clip on the side of the processing device is arranged to be shorter as the desirable number of the plural cables decreases.

In the detecting device according to the invention as set forth in claim 4, the detectors are antennas, the processing device is a receiving device, each of the antennas is arranged at a desirable position on an outside-body of a subject and receives a radio wave transmitted from a body-insertable device, and the coaxial cables are arranged along the outside-body of the subject.

A manufacturing method of a detecting device in which a plurality of detectors and a processing device which receives and processes a detection signal from the detectors are connected through a plurality of cables, according to claim 5, includes a connecting step of connecting the detectors and the cables; a clipping step of forming into a flat cable the plurality of cables to which the detectors are connected at the connecting step, with cable clips at predetermined intervals so that each cable clip bundles more than one cable; and a connector connecting step of connecting a connector to ends of the plurality of cables which are bundled at the clipping step, the ends being on a side of the processing device.

In the manufacturing method of a detecting device according to the invention as set forth-in claim 6, at the clipping step, a number of the cables formed into the flat cable is decreased in stages from the side of the processing device to make the cables branch off in stages.

In the manufacturing method of a detecting device according to the invention as set forth in claim 7, the predetermined intervals are set shorter as the number of the cables decreases.

### EFFECT OF THE INVENTION

According to the detecting device and the manufacturing method of the present invention, since plural and independent cables that connect between each detector and a processing device are aligned in a single row such that the cables contact each other and that movement of each of the cables is limited with cable clips, to be formed into a flat cable, it is possible to suppress scattering of each of the cables similarly to a case of using flat cables, while allowing portions of the cables other than portions clipped with the cable clips to be flexibly moved. Therefore, such effects can be obtained that lateral movement of a cable group in which a plurality of cables are bundled becomes flexible, and flexibility of the detectors at the time of mounting and after mounting can be sufficiently secured.

### BRIEF DESCRIPTION OF DRAWINGS

FIG. 1 is a schematic diagram showing an entire configuration of a radio in-vivo information acquiring system that includes a receiving device according to an embodiment of the present invention;
FIG. 2 is a block diagram showing a configuration of the receiving device shown in FIG. 1;
FIG. 3 is a diagram showing a configuration of coaxial cables and receiving antennas that are connected to a radio unit;
FIG. 4 is a diagram for explaining intervals of cable clips;
FIG. 5 is a diagram showing a state of the coaxial cables shown in FIG. 3 when the cables are bent;
FIG. 6 is a diagram showing a state in which the coaxial cables shown in FIG. 3 are wired on a subject;
FIG. 7 is a diagram showing a modified example of the coaxial cables shown in FIG. 3; and
FIG. 8 is a diagram showing a manufacturing method of the coaxial cables shown in FIG. 3.

### EXPLANATIONS OF LETTERS OR NUMERALS

- 1: SUBJECT
- 2: RECEIVING DEVICE
- 2a: RADIO UNIT
- 2b: MAIN RECEIVING UNIT
- 3: CAPSULE ENDOSCOPE
- 4: DISPLAY DEVICE
- 5: PORTABLE RECORDING MEDIUM
- 10: COAXIAL CABLE GROUP
- 10-1 to 10-8: COAXIAL CABLES
- 10a: CONNECTOR
- 11.: RECEIVING CIRCUIT
- 12: SIGNAL PROCESSIGN CIRCUIT
- 13: A/D CONVERTER
- 14: DISPLAY UNIT
- 15: STORAGE UNIT
- 16: POWER SUPPLY UNIT
- 20a to 20d: CABLE CLIP
- C1: CONTROL UNIT
- Ca: SWITCHING CONTROLLER
- SW: CHANGEOVER SWITCH
- A1 to An, A11 to A18: RECEIVING ANTENNAS

### BEST MODE(S) FOR CARRYING OUT THE INVENTION

A radio in-vivo information acquiring system including a receiving device as a detecting device according to best modes for carrying out the invention will be explained below.

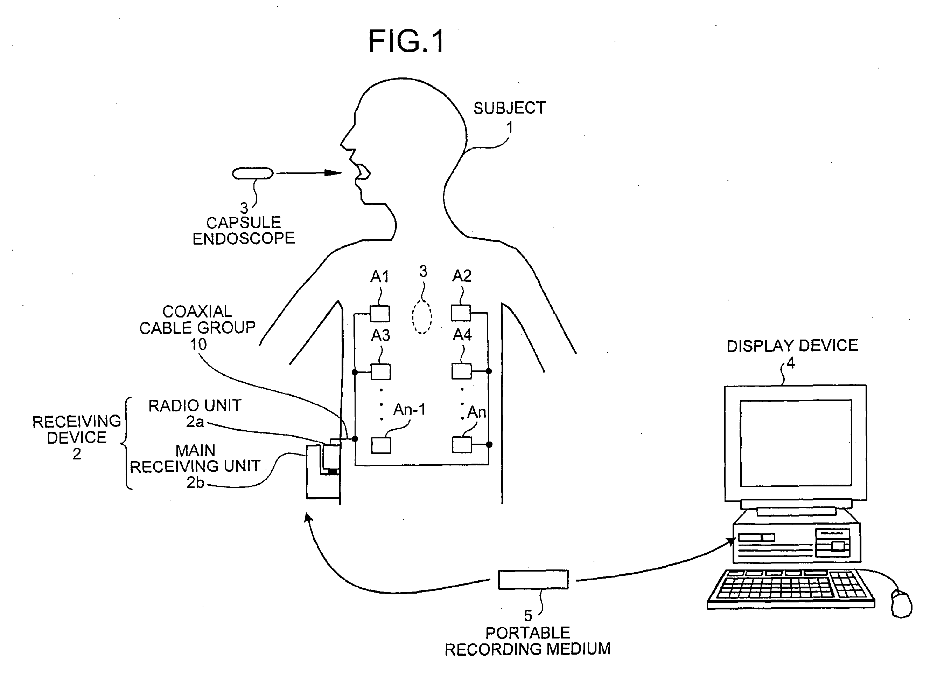

FIG. 1 is a schematic diagram showing an entire configuration of a radio in-vivo information acquiring system. In this radio in-vivo information acquiring system, a capsule endoscope is used as an example of a body-insertable device. As shown in FIG. 1, the radio in-vivo information acquiring system includes a capsule endoscope 3 that is inserted into the body of a subject 1, and that acquires images inside of body cavities to perform data transmission of an image signal and the like to a receiving device 2 by radio communication, the receiving device 2 that receives the data of the images inside of body cavities that is transmitted by radio communication from the capsule endoscope 3, a display device 4 that displays the image inside of body cavities based on the image signal received by the receiving device 2, and a portable recording medium 5 to communicate the data between the receiving device 2 and the display device 4. Moreover, the receiving device 2 includes a radio unit 2a that includes a plurality of receiving antennas A1 to An that are attached on an outer surface of the body of the subject 1, and a main receiving unit 2b that performs a process and the like of the radio signal that is received through the receiving antennas A1 to An. These units are detachably connected through a connector and the like. Each of the receiving antennas A1 to An can be installed on a jacket that can be worn by the subject 1, and the receiving antennas A1 to An can be mounted by putting this jacket on the subject 1. In this case, the receiving antennas A1 to An can be attachable and detachable to the jacket. The radio unit 2a is connected with the receiving antennas A1 to An with a coaxial cable group 10. The receiving antennas A1 to An correspond to the detectors, a main unit of the radio unit 2a and the main receiving unit 2b correspond to the processing device, and the receiving device 2 corresponds to the detecting device.

The display device 4 is to display the images inside of body cavities acquired by the capsule endoscope 3, and is realized with a workstation and the like to perform image display based on the data obtained by the portable recording medium 5. Specifically, the display device 4 can take a configuration to directly display the images with a CRT display, a liquid crystal display, and the like, or can take a configuration to output the images to another medium such as a printer.

The portable recording medium 5 is implemented with the CompactFlash (registered trademark) memory or the like, and is attachable and detachable to the main receiving unit 2b and the display device 4, and has a function enabling output and record of information when attached to those. Specifically, the portable recording medium 5 is attached to the main receiving unit 2b, during the capsule endoscope 3 is moving inside of body cavities of the subject 1, to store the data transmitted from the capsule endoscope 3. The portable recording medium 5 is configured to be detached from the main receiving unit 2b and then attached to the display device 4 after the capsule endoscope 2 is discharged out of the subject 1, in other words, after imaging of the inside of the subject 1 is completed, and the data stored therein is read out by the display device 4. By performing data communication between the main receiving unit 2b and the display device 4 with the portable recording medium 5, the subject 1 can freely act even while imaging inside the body cavities, and it is possible to contribute to shortening a period of the data communication with the display device 4. For the data communication with the display device 4, another recording device equipped in the main receiving unit 2b can be used, and the recording device can be configured to be connected to the display device 4 by wired connection or by wireless connection.

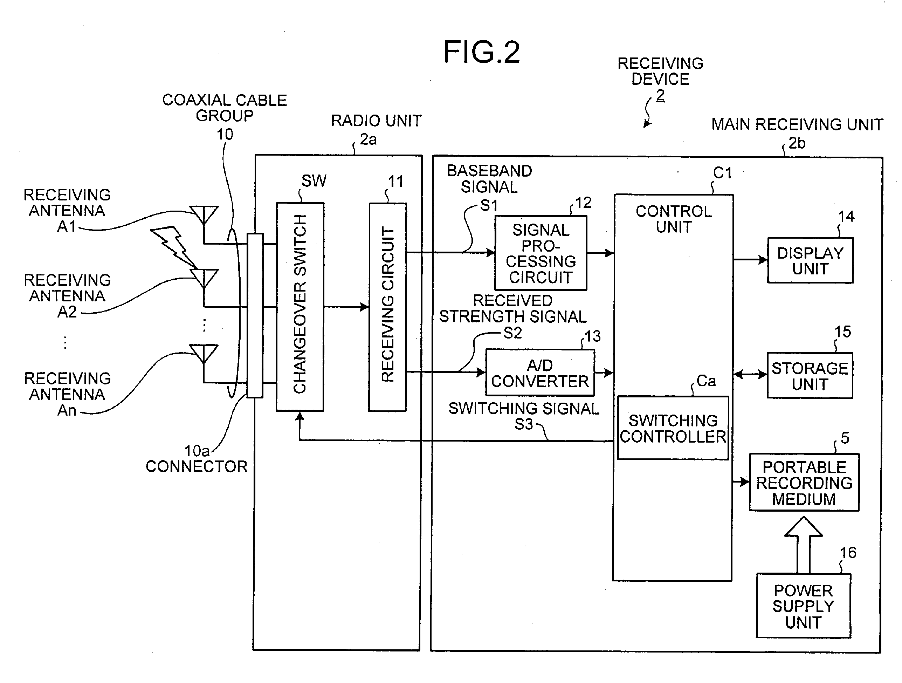

FIG. 2 is a block diagram showing a configuration of the receiving device 2 shown in FIG. 1. The radio unit 2a receives the radio signal transmitted from the capsule endoscope 3 and decodes into a baseband signal. As shown in FIG. 2, the radio unit 2a includes a changeover switch SW to which the coaxial cable group 10 connecting the receiving antennas A1 to An is connected through a connector 10a and that performs a connection switching process to selectively switch to any one of the receiving antennas A1 to An, and a receiving circuit 11 that is connected at a subsequent stage of the changeover switch SW and that amplifies to decode the radio signal from the receiving antennas A1 to An that are switched to be connected by the changeover switch SW.

The main receiving unit 2b receives to process the baseband signal decoded by the radio unit 2a. As shown in FIG. 2, the main receiving unit 2b includes a signal processing circuit 12 and an A/D converter 13 that are connected at a subsequent stage of the receiving circuit 11, a display unit 14 that displays the image data processed by the signal processing circuit 12, a storage unit 15 that stores various kinds of information, the portable information recording medium 5, a control unit C that controls each of these components, and a power supply unit 16 that supplies power to the main receiving unit 2b and the radio unit 2a. The control unit C includes a switching controller Ca that performs a switching control of the antennas.

The receiving circuit 11 amplifies the radio signal output from the changeover switch SW, outputs a decoded baseband signal S1 to the signal processing circuit 12, and outputs a received strength signal S2 that indicates signal strength of the amplified radio signal to the A/D converter 13. The image data processed by the signal processing circuit 12 is stored in the portable information recording medium 5 by the control unit C, and images are displayed on the display unit 14 as necessary. The received strength signal S2 converted into a digital signal by the A/D converter 13 is input to the control unit C. The switching controller Ca selects a receiving antenna that has received at the highest signal strength as a receiving antenna to acquire the image data based on this received strength signal S2 obtained by sequentially switching the receiving antennas A1 to An, and outputs a switching signal S3 instructing switch to this antenna, to the changeover switch SW. Moreover, the control unit C stores the signal strength received by each of the receiving antennas together with the image data in an associated manner in the portable information recording medium 5. The store signal strength of each of the receiving antennas is used as information to calculate a position of the capsule endoscope 3 inside the body at the time of reception of the image data.

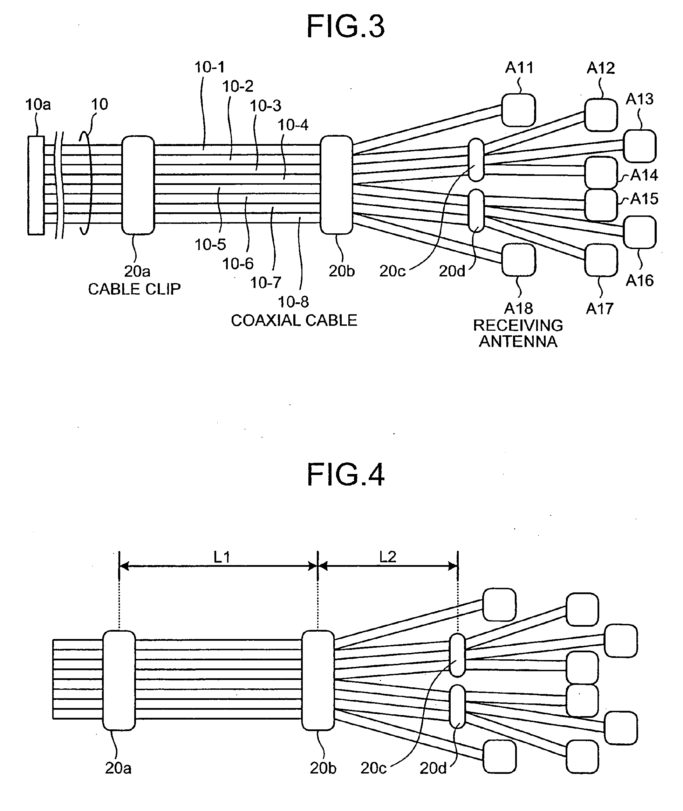

With reference to FIG. 3, a structure of the coaxial cable group connecting the receiving antennas A1 to An will be explained. In this example, a case in which eight units of receiving antennas A11 to A18 are connected to the radio unit 2a is explained. As shown in FIG. 3, the eight receiving antennas A11 to A18 are connected to coaxial cables 10-1 to 10-8 respectively. The connector 10a is connected on a side of the radio unit 2a. Cable clips 20a and 20b bundle the eight coaxial cables 10-1 to 10-8 in parallel, and cable clips 20c and 20d bundle three pieces of the coaxial cables 10-2 to 10-4, and 10-5 to 10-7 respectively in parallel on a side of the receiving antennas A11 to A18. Since the receiving antennas A11 to A18 are mounted at various positions on the subject 1, a length of each of the coaxial cables 10-1 to 10-8 varies. If there are receiving antennas to be arranged in an identical direction, such receiving antennas are bundled by the cable clips 20a to 20d, the number of cables to be bundled decreases as being away from the radio unit 2a, and thus, each of the coaxial cables 10-1 to 10-8 branches off. Each of the coaxial cables 10-1 to 10-8 is independent cable, and the coaxial cables 10-1 to 10-8 between the cable clip 20a and the cable clip 20b, or between the cable clip 20a and the connector 10a usually just contact each other.

It is preferable that each interval between the cable clips 20a and 20b, or between the cable clip 20a and the connector 10a is determined depending on the number of the coaxial cables 10-1 to 10-8 bundled with the cable clips 20a to 20d, and as the number of the coaxial cables 10-1 to 10-8 to be bundled increases, a larger interval is set. For example, as shown in FIG. 4, an interval L2 between the cable clip 20b and each of the cable clips 20c and 20d that bundle three pieces of the coaxial cables 10-2 to 10-5 and the coaxial cables 10-6 to 10-8 is set smaller than an interval L1 between the cable clips 20a and 20b. that bundle eight pieces of the coaxial cables 10-1 to 10-8. This is because if the coaxial cables bundled are few, flexibility between the coaxial cables increases, thereby decreasing bundling effect of the cable clips.

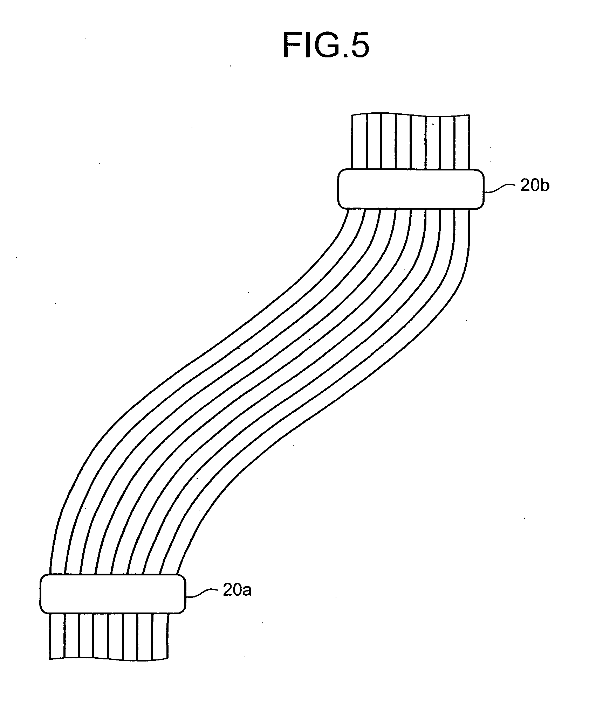

In the present embodiment, unlike flat cables, flexibility of the each of the coaxial cables is high, and therefore, such cable wiring is possible that the cables are bent while maintaining the cable clips 20a and 20b in parallel, as shown in FIG. 5.

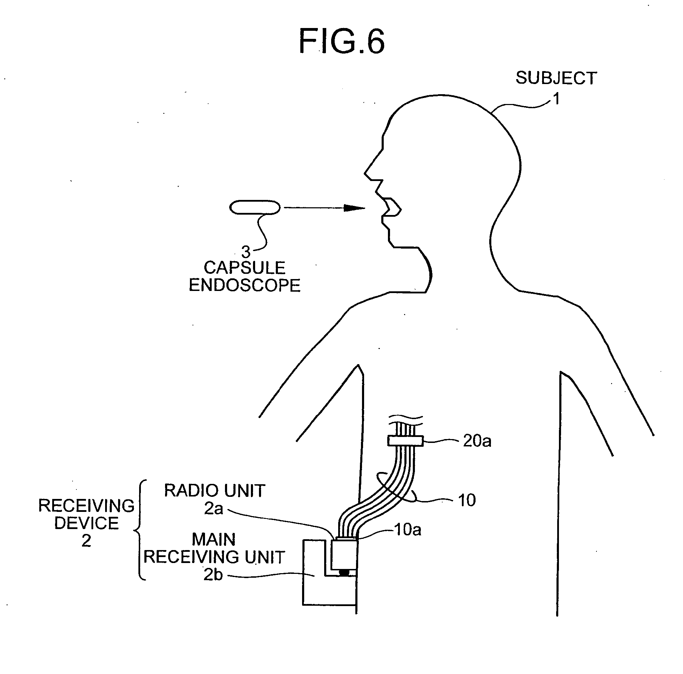

Therefore, the cable wiring as shown in FIG. 6 is possible on the subject 1, and highly flexible wiring is achieved. If this wiring is applied with a flat cable, deflection is caused inside the bend, thereby causing large stress on the connector 10a of the radio unit 2a. However, if the coaxial cable group 10 and the cable clips 20a to 20d described in the present embodiment are applied, the stress on each coaxial cable is small and uniform, and no large stress is applied on the radio unit 20a. Accordingly, resistance of the receiving device 2 is improved.

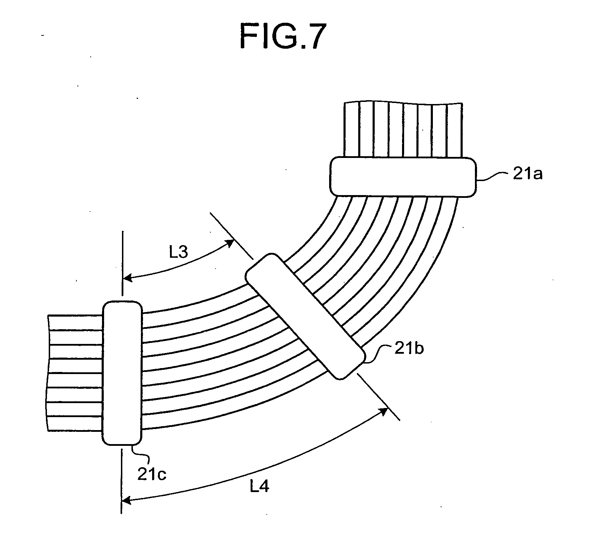

By using this coaxial cable group 10 and the cable clips 20a to 20d, a free shape cable can be manufactured that is difficult to be manufactured with an ordinary flat cable. For example, the coaxial cable group having a desirable curvature as shown in FIG. 7 can be easily formed into a flat cable. Furthermore, by making intervals of the cable clips extremely small, it is possible to obtain strength close to that of the flat cable.

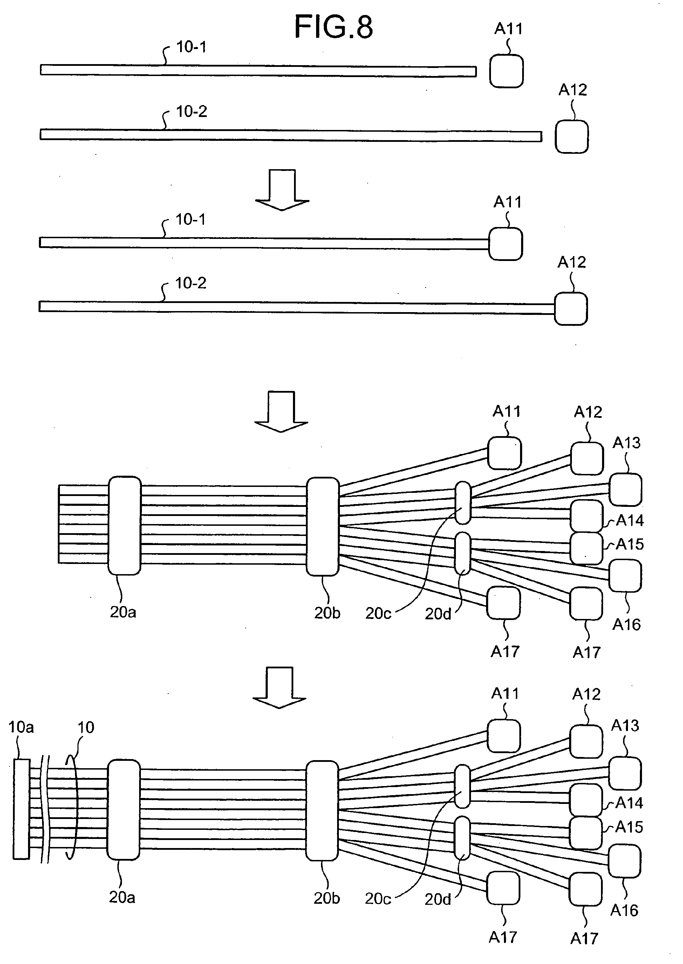

FIG. 8 is a diagram for explaining a manufacturing method of the detecting device according to the embodiment of the present invention. In the manufacturing of this detecting device, first, the coaxial cables 10-1 to 10-8 each having a length corresponding to each of the receiving antennas A11 to A18 and the receiving antennas A11 to A18 are connected respectively as shown in FIG. 8.

Subsequently, considering a positional relationship of each of the receiving antennas A11 to A18, the coaxial cables 10-1 to 10-8 are bundled with the cable clips 20a to 20d. Ends of the coaxial cables 10-1 to 10-8 on a side of the radio unit 2a are cut to be trued up, to be connected to the connector 10a.

By applying such a manufacturing method, the receiving antennas A11 to A18 and the coaxial cables 10-1 to 10-8 can be connected with ease, and even if connection failure occurs between each of the receiving antennas and the coaxial cable, only the coaxial cable between this receiving antenna and the coaxial cable should be replaced, thereby improving a yield. When the receiving antennas A11 to A18 are put with the conventional flat cable, since the receiving antennas A11 to A18 are connected to flat cables having various lengths, it takes long time for manufacturing, and it has not been able to improve a yield.

Moreover, in this manufacturing method, a set of each receiving antenna and a coaxial cable is manufactured first, and therefore, it is possible to form into a flat cable corresponding to the desirable number of the coaxial cables, and to easily realize a receiving device using only one set of the receiving antenna and the coaxial cable.

While in the above embodiment, an example of forming the coaxial cables into a flat cable has been explained, it is not limited thereto, and it is possible to similarly apply to a cable of a single lead.

### INDUSTRIAL APPLICABILITY

As described above, the detecting device and the manufacturing method of the detecting device are useful for securing flexibility at the time of mounting and after mounting detectors, such as antennas, that are disposed at desirable portions on the outside-body of a subject, and particularly suitable for a receiver of a capsule endoscope.

## Claims

1. A detecting device in which a plurality of detectors and a processing device which receives and processes a detection signal from the detectors are connected through a plurality of cables, comprising
a cable clip that forms the plurality of respective cables into a flat cable so that the cables which are placed parallel to and in contact with each other are restrained from moving at desirable positions from a side of the processing device.

2. The detecting device according to claim 1, wherein
the cables are coaxial cables, and
the cable clip is arranged so as to make the coaxial cables branch off in stages by decreasing a number of the coaxial cables which is formed into the flat cable in stages from the side of the processing device.

3. The detecting device according to claim 1 or 2, wherein a length of the cables formed into the flat cable with the cable clip on the side of the processing device is arranged to be shorter as the desirable number of the plural cables decreases.

4. The detecting device according to any one of claims 1 to 3, wherein
the detectors are antennas,
the processing device is a receiving device,
each of the antennas is arranged at a desirable position on an outside-body of a subject, and receives a radio wave transmitted from a body-insertable device, and
the coaxial cables are arranged along the outside-body of the subject.

5. A manufacturing method of a detecting device in which a plurality of detectors and a processing device which receives and processes a detection signal from the detectors are connected through a plurality of cables, comprising:
a connecting step of connecting the detectors and the cables;
a clipping step of forming into a flat cable the plurality of cables to which the detectors are connected at the connecting step, with cable clips at predetermined intervals so that each cable clip bundles more than one cable; and
a connector connecting step of connecting a connector to ends of the plurality of cables which are bundled at the clipping step, the ends being on a side of the processing device.

6. The manufacturing method of a detecting device according to claim 5, wherein at the clipping step, a number of the cables formed into the flat cable is decreased in stages from the side of the processing device to make the cables branch off in stages.

7. The manufacturing method of a detecting device according to claim 5 or 6, wherein the predetermined intervals are set shorter as the number of the cables decreases.
